# FASCICULE DE BREVET EUROPEEN

(11) **EP 0 579 596 B1**
(45) Date de publication et mention de la délivrance du brevet: **27.12.1995**
(21) Numéro de dépôt: 91903578.2
(22) Date de dépôt: 18.02.1991
(51) Int. Cl.: C12N 5/00, C12N 11/08, C12M 3/02

(54) **SUPPORT POUR LA CULTURE DE CELLULES ET PROCEDE DE PREPARATION D'UN TEL SUPPORT**
TRÄGER ZUM ZÜCHTEN VON ZELLEN UND VERFAHREN ZU SEINER HERSTELLUNG
CELL CULTURE MEDIUM AND METHOD FOR PREPARING SUCH MEDIUM

(43) Date de publication de la demande: 26.01.1994
(73) Titulaire: COMPUTER CELL CULTURE CENTER S.A., 7000 Mons (BE)
(72) Inventeur: MILLER, Alain, B-7000 Mons (BE); DE HOLLAIN, Guy, F-78540 Vernouillet (FR); MASQUELIER, Dominique, B-1030 Bruxelles (BE)
(74) Mandataire: Plougmann, Vingtoft & Partners A/S
(86) Numéro de dépôt international: BE9100012
(87) Numéro de publication internationale: WO9214814

(56) Documents cités:
- EP-A- 0 303 294
- LU-A- 86 337
- US-A- 4 266 032

## Description

La présente invention est relative à un support pour la culture de cellules ancrage-dépendantes (CAD), en forme de microporteurs en un matériau biocompatible. Elle concerne également un procédé de préparation d'un tel support.

Les cellules de mammifères, utilisées en tant que telles, pour la production de vaccins humains et vétérinaires, pour l'obtention de molécules à très haute valeur ajoutée ou d'autres productions analogues, sont généralement des CAD : elles nécessitent un support pour croître. En l'absence de celui-ci, ces cellules, en suspension, dégénèrent et meurent.

La culture en masse est d'autant mieux réalisée industriellement que la surface offerte à l'accrochage est grande par rapport au volume total du support utilisé. En calculant le rapport surface/volume de différents supports, on constate que ce rapport peut varier entre 1,25 et 150,0 pour certains microporteurs actuels (voir M. BUTLER, Growth Limitations in Microcarrier Cultures, in : Advances in Biochemical Engineering/Biotechnology, pages 57 et suivantes, volume 34, Springer Verlag, 1987).

Industriellement, il est plus facile de façonner des microporteurs à géométrie sphérique : les microbilles. C'est la raison pour laquelle l'utilisateur a le choix aujourd'hui d'une très grande variété de ce type de microporteur (voir entre autres, A. SHAHAR et cons., Nerve and Muscle Cells on Microcarriers in Culture, in : Advances...., op.cit., pages 33 et suivantes, volume 34, 1987).

Malgré toutes leurs potentialités, il n'existe que peu d'exemples au monde où les CAD soient cultivées en masse sur des microporteurs. Les boîtes de Roux, les flacons rotatifs et autres "usines à cellules" restent les moyens les plus généralement utilisés.

Les raisons qui sous-tendent cette situation ainsi que les moyens à mettre en oeuvre, nécessaires pour y mettre fin, ont fait l'objet d'une étude détaillée (voir A.O.A. MILLER et cons., Microbeads and Anchorage-Dependent Eukaryotic Cells : The Beginning of a New Era in Biotechnology, in : Advances...., op.cit., pages 73 et suivantes, volume 39, 1989).

Récemment, dans le but essentiellement de diminuer les coûts de production des substances produites par les CAD, il a été fait usage de spirales en polystyrène, et de disques de 1,5 mm d'épaisseur et de 6 mm de diamètre formés d'un treillis de polypropylène enrobé d'un film de polyester (voir A. KADOURI et cons., Polystyrene substratum for bulk culture of anchorage dependent cells, in Cytotechnology, 1 : 301-307, 1988 ; M. NEEMAN et cons., Adaptation of Culture Methods for NMR Studies of Anchorage-Dependent Cells, in Magnetic Resonance in Medecine, 7, 236-242, 1988 ; A. KADOURI et cons., Production of Anti-Leukemic Factor from Stroma Cells in a Stationary Bed Reactor on a New Cell Support, in 9th ESACT Meeting, Belgium, pages 327 et suivantes, Butteworths, 1989).

Tous les supports précités se caractérisent par une géométrie tridimensionnelle. Pour beaucoup d'entre eux, même en ce qui concerne les microbilles et les disques épais en matière tissée, le rapport surface/volume du support reste peu favorable. Ces disques épais en treillis de matière tissée présentent d'ailleurs l'inconvénient de permettre la pénétration et l'accrochage de cellules à l'intérieur des disques. Ces cellules ne peuvent plus ou très difficilement être alors séparées de leur support, ce qui représente un grand inconvénient et une perte sèche lorsqu'on cultive les cellules pour elles-mêmes. En fait, ces disques permettent uniquement une culture de cellules accrochées ad vitam à leur support, par exemple en vue de la sécrétion d'une substance donnée que l'on récolte. Dans ce type de culture, les supports de cellules ne sont pas en suspension.

La présente invention a pour but de résoudre les problèmes posés par les supports selon la technique antérieure, tout en améliorant notablement le rapport surface/volume du support de culture. L'invention doit permettre de préférence un détachement aisé des cellules à partir de leur support, sans traitement endommageant les cellules, ainsi qu'une culture en suspension. Il est nécessaire que les supports suivant l'invention soient biocompatibles. Il est préférable qu'ils permettent une analyse aisée des cellules au cours de leur croissance, et que leur densité permette une culture en suspension dans le milieu de culture. Il est avantageux de parvenir à ce que les supports résistent aux conditions de stérilisation.

Pour résoudre ces problèmes, on a prévu suivant l'invention un support pour la culture des CAD, en forme de microporteurs en un matériau biocompatible, dans lequel les microporteurs présentent une géométrie bidimensionnelle et comportent deux faces d'ancrage apposées avec une épaisseur inférieure ou égale à 35 microns, faces sur chacune desquelles les cellules peuvent s'accrocher et se développer, sans pénétration possible des cellules entre ces deux faces.

Par géométrie bidimensionnelle, il faut entendre que l'épaisseur de ces microporteurs tant à devenir infime et négligeable par rapport aux dimensions des CAD. Cette réduction d'épaisseur est telle qu'il n'y a aucune possibilité de croissance de cellules au sein du support, mais uniquement sur ses deux faces d'ancrage.

Suivant une forme de réalisation de l'invention les microporteurs ont une épaisseur de préférence inférieure ou égale à 25 microns.

Suivant une forme préférée de réalisation de l'invention, les microporteurs ont une forme discoïdale, et en particulier une forme de disques ou de fuseaux. Le terme discoïdal désigne, dans le cadre de la présente invention, la famille de particules obtenues par l'intersection orthogonale (disques) ou oblique (fuseaux) de deux plans parallèles séparés l'un de l'autre d'une distance de 35 microns au maximum, avec un cylindre plein.

Suivant une forme avantageuse de réalisation de l'invention, les microporteurs ont, dans le milieu de culture, une densité comprise entre 0,90 et 1,10 g/cm³, de préférence entre 1,03 et 1,05 g/cm³. La densité des microporteurs est appropriée en particulier pour que, dans le fluide servant de milieu de culture, les turbulences engendrées pour les maintenir en suspension ne traumatisent pas les cellules accrochées et n'altèrent pas leur viabilité. Trop légers, les microporteurs flottent, trop lourds, ils sédimentent au fond du bioréacteur. Leur maintien en suspension nécessite donc des conditions de vitesses d'agitation du milieu de culture telles que les forces de cisaillement ainsi engendrées ne décrochent pas les cellules, en leur infligeant de multiples traumatismes.

Suivant une forme de réalisation particulière de l'invention, les microporteurs sont en un matériau résistant à la stérilisation. Cela permet d'éviter que des contaminants (bactéries, mycoplasmes, levures, virus, etc....) adhérant aux microporteurs ne se développent dans la culture de cellules au détriment de ces dernières.

Suivant une autre forme de réalisation de l'invention, les microporteurs sont transparents pour des ondes comprises entre 400 nm et 1000 nm. Par transparence, on entend que, dans ce domaine de longueurs d'ondes, la lumière traverse les microporteurs sans atténuation importante de l'intensité du faisceau lumineux émergeant par rapport à l'intensité du faisceau de lumière incident. Une absorption inférieure à 1% est considérée comme tout à fait avantageuse.

Suivant une forme de réalisation de l'invention, les microporteurs sont en une matière polymère. On peut par exemple envisager, comme matière polymère, un polymère présentant de nombreux groupes aromatiques, et en particulier du polystyrène. D'autres matières ne sont bien entendu pas à exclure et par exemple des disques en matière hydrophile, comme de la cellophane ou du poly-D-hydroxybutyrate, sont concevables. Ils présentent cependant l'inconvénient de gonfler à l'eau, ce qui risque de leur faire perdre leur géométrie bidimensionnelle.

Il est nécessaire, suivant l'invention, que les microporteurs soient biocompatibles. Par biocompatibilité, il faut entendre, dans le sens de la présente invention, que les CAD sont capables de s'accrocher sur les faces des microporteurs suivant l'invention et de s'y multiplier.

Il est avantageusement prévu dans ce but de revêtir les deux faces des microporteurs suivant l'invention d'un mince dépôt métallique. Ce dépôt est avantageusement à base de titane. On peut envisager également le platine par exemple.

Les microporteurs suivant l'invention offrent une série d'avantages significatifs par rapport aux microporteurs tridimensionnels utilisés aujourd'hui.

Un calcul simple permet de se rendre compte que la surface totale de deux disques infiniment minces dont le diamètre est égal à celui de la sphère à l'intérieur de laquelle ils s'inscrivent (disques "équatoriaux") est égale à la surface extérieure de la sphère (= microbille).

A l'intérieur des deux calottes ainsi aménagées au-dessus et en dessous de ces deux disques équatoriaux, peuvent se disposer d'autres disques dont le diamètre cette fois va en diminuant au fur et à mesure qu'on s'éloigne de l'équateur pour se diriger vers les pôles. Adoptant une épaisseur de 10 microns pour l'ensemble des disques ainsi engendrés et exprimant leur surface en terme de "surface équatoriale équivalente", la surface totale ainsi fournie par les disques est 5 à 7 fois supérieure à la surface extérieure de la sphère.

Bien sûr, en suspension, les mouvements aléatoires de ces microdisques ainsi que l'augmentation d'épaisseur résultant de l'adhésion des CAD concourent à diminuer la surface totale disponible. Néanmoins celle-ci reste supérieure à au moins deux fois la surface externe de la sphère.

Par ailleurs, la réduction du diamètre des microdisques jusqu'à par exemple 80 microns permet d'utiliser les ajutages développés en cytométrie de flux pour réaliser la focalisation hydrodynamique. Le défilement à la file indienne dans le faisceau du laser interrogateur peut encore être amélioré en diminuant les degrés de liberté des microdisques consécutivement à leur façonnage en supports fusiformes. Une telle approche permettrait d'appliquer aux cellules ancrées sur le support les techniques d'analyse et de tri développées en cytométrie de flux, sans qu'il soit nécessaire de les en détacher au préalable par la trypsine ou le tétraacétate d'éthylènediamine (EDTA). L'analyse in situ devient aisée et les endommagements cellulaires résultant d'un traitement à la trypsine ou au EDTA sont évités.

Selon leur épaisseur on pourra envisager des microporteurs minces, de préférence de 1 à 25 microns, ou des microporteurs ultra-minces, c'est-à-dire d'une épaisseur inférieure à 1 micron.

Les microdisques suivant l'invention présentant un diamètre inférieur à 500 microns présentent l'énorme avantage de pouvoir être transférés par pipettage et ils peuvent être utilisés dans des cultures en suspension, sans problème. Les microdisques, dont le diamètre est d'une dimension supérieure, bien qu'ils puissent également être utilisés en suspension, de par le fait qu'ils ne peuvent être transférés que par transvasement, trouvent leur utilisation plutôt dans les cultures immobilisées.

La présente invention concerne également un procédé de fabrication d'un support pour la culture de CAD, en forme de microporteurs en un matériau biocompatible, ce procédé étant caractérisé en ce qu'il comprend un découpage d'un film mince ou ultra-mince en microporteurs présentant une géométrie bidimensionnelle et comportant deux faces d'ancrage apposées avec une épaisseur inférieure ou égale à 35 microns, faces sur chacune desquelles les cellules peuvent s'accrocher et se développer, sans pénétration possible des cellules entre ces deux faces.

Le découpage du film mince ou ultra-mince peut être effectué d'une manière quelconque appropriée, selon la nature du film. Les films de polystyrène par exemple présentent de nombreux groupes aromatiques et ils se prêtent à la photoablation par laser excimère. On peut aussi envisager un découpage mécanique, à l'emporte-pièce, particulièrement avantageux dans le cas de microporteurs de grand diamètre. Malgré la relativement mauvaise qualité de ce type de découpe, il est possible d'envisager une découpe par pyrolyse (laser à argon), par exemple de disques en cellophane préalablement colorée en rouge avec une substance non toxique. D'autres procédés sont bien sûr éventuellement envisageables.

Dans le cas où le matériau dont est fait le support suivant l'invention n'est pas en soi biocompatible, ce qui est le cas par exemple du polystyrène, il faut prévoir en outre, avant le découpage, un traitement approprié pour rendre ce matériau biocompatible.

Dans ce but, on prévoit, suivant l'invention une application d'un mince dépôt métallique, en particulier à base de titane, sur chacune des surfaces du film mince.

Un procédé de vaporisation d'une mince couche de titane sur la surface interne d'une boîte de Petri est décrit notamment dans le brevet LU-A-86337. On peut envisager d'autres procédés d'application, notamment par projection de plasma de titane à l'aide d'un magnétron.

L'utilisation de disques ou fuseaux minces en polystyrène ou en cellophane revêtus d'une couche de titane sur les deux faces permet d'envisager un décrochage électrolytique des cellules.

Pour rendre biocompatible le film mince ou ultra-mince on peut aussi envisager, suivant l'invention, de faire passer une décharge électrique à effet couronne au travers du film.

Il faut noter que, si le polystyrène ne résiste pas à une stérilisation à l'autoclave, il est possible de le stériliser par irradiation aux rayons gamma. Une stérilisation à l'alcool peut aussi être envisagée.

Des essais destinés à évaluer la capacité d'adhésion de CAD ont été réalisés sur des disques de 8 mm de diamètre, découpés à l'emporte-pièce dans des films de polystyrène et de cellophane.

Les cellules utilisées pour ces essais sont des cellules Véro, disponibles sur le marché, qui ont été cultivées en boîtes de Roux de 200 cm² de surface dans un incubateur humide (37°C, 95% air/ 5% CO₂) dans du milieu 199 (Gibco) enrichi par 5% de sérum de veau foetal (Gibco) et contenant 100 U de pénicilline et 0,1 mg de streptomycine/ml.

Le fond des boîtes contenant les cellules est rincé par une solution de PBS et les cellules sont détachées par trypsinisation (solution de trypsine Gibco 1/250 à 0,5% contenant 0,2% de EDTA). Cette digestion enzymatique de la matrice est réalisée jusqu'au moment où l'on peut voir les cellules se décrocher à l'oeil nu. A ce moment, l'activité de l'enzyme est arrêtée par l'addition de milieu complet. Les cellules sont centrifugées (1000 rpm-centrifugeuse ECCO type H 6,5) pendant 10 minutes. Le surnageant éliminé, les cellules sont resuspendues dans un volume déterminé de milieu afin de déterminer leur concentration, en utilisant à cet effet la cellule de comptage de Burker.

L'incubation de 40.000 cellules est ensuite réalisée à la surface des microporteurs suivant l'invention, préalablement recouverts ou non de titane. Ces microporteurs ont été déposés dans une boîte multipuits (24). Au fond des puits, ils sont maintenus plaqués par une goutte de glycérol. Les observations sont réalisées 24 heures après cette inoculation.

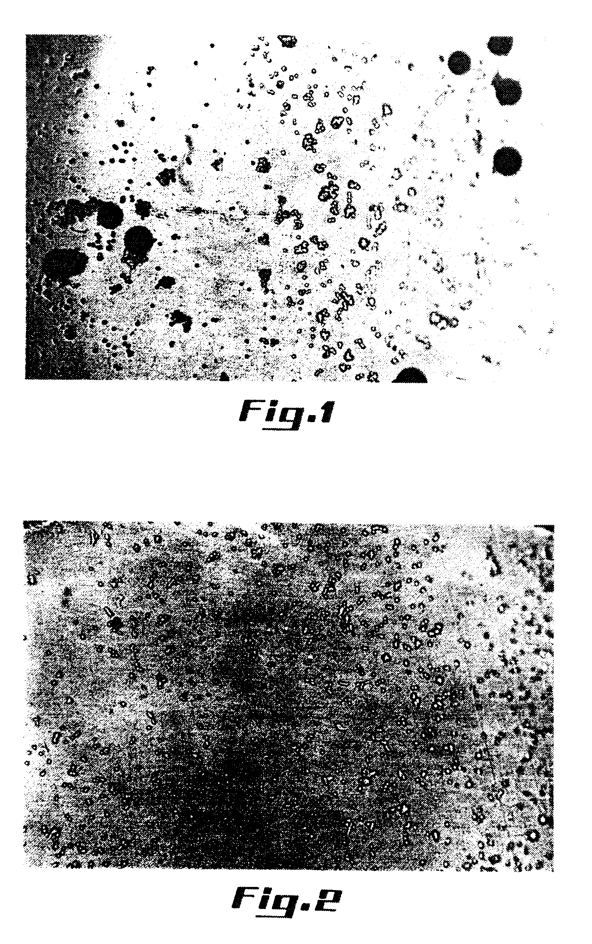

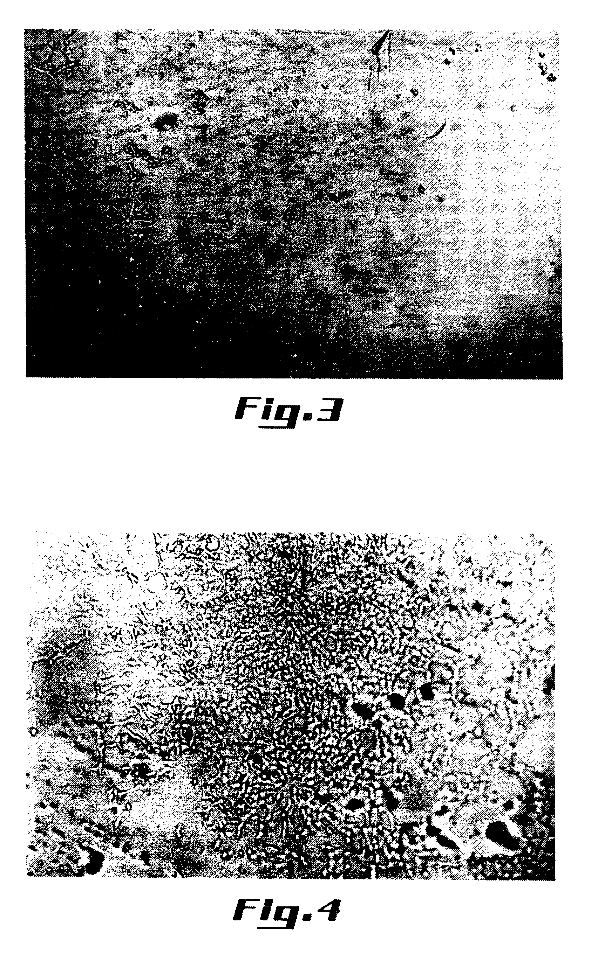

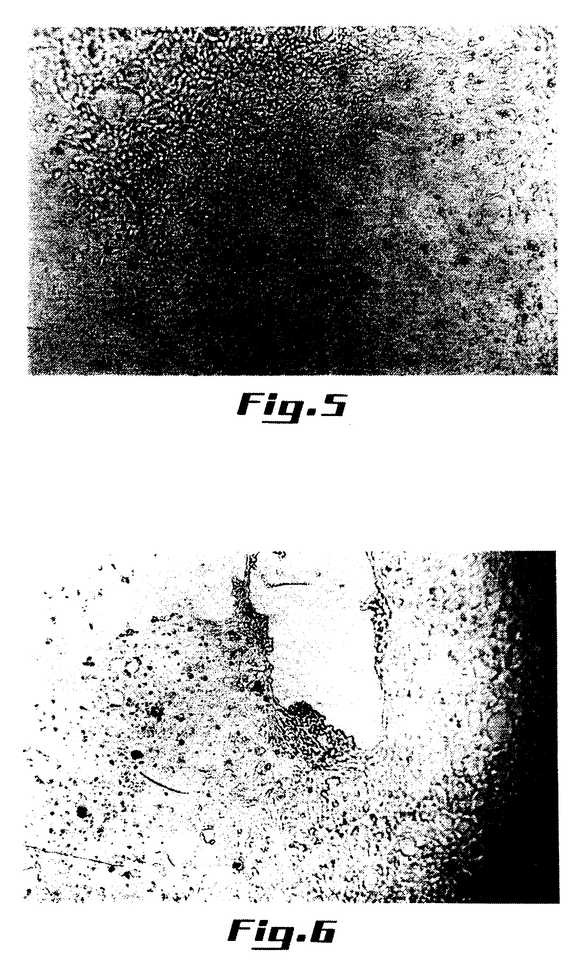

Les figures 1 à 3 représentent des photos prises au microscope de trois disques témoins réalisés à partir de films respectivement en cellophane de qualité A, cellophane de qualité B et polystyrène, et soumis aux essais décrits ci-dessus.

Les figures 4 à 6 représentent des photos semblables de trois disques réalisés à partir des mêmes films que les figures 1 à 3, mais après projection au magnétron d'un plasma de titane sur leurs deux faces.

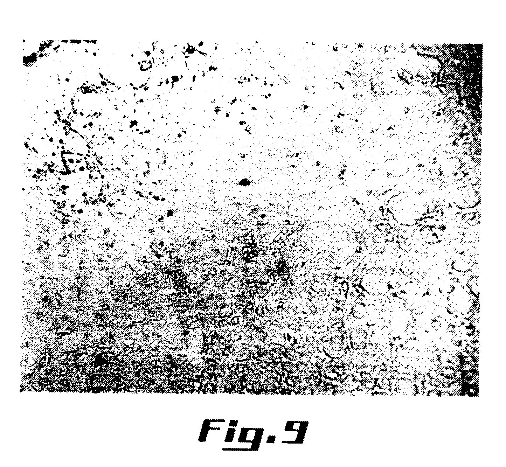

Les figures 7 à 9 représentent des photos semblables de trois disques réalisés à partir des mêmes films que pour les figures 1 à 3, mais après vaporisation de titane sur leurs deux faces.

Il ressort de ces figures, que les disques utilisés suivant l'invention connaissent un rendement cellulaire de loin supérieur après application d'une couche de titane sur leurs deux faces.

Il est évident que, si le film découpé est en une matière biocompatible en soi, ce qui peut être le cas de certains types de cellophane par exemple, l'application d'une couche mince de titane devient superflue.

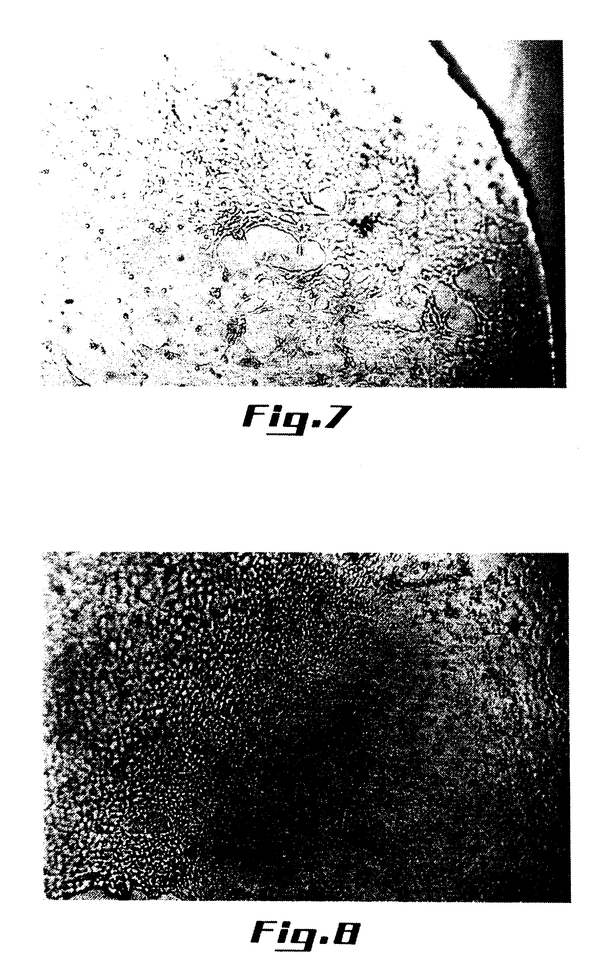

Un essai semblable aux précédents a été réalisé sur des disques découpés dans une feuille de polystyrène traitée par une décharge à effet couronne, pour la rendre biocompatible. Les résultats obtenus sont aussi satisfaisants que ceux illustrés sur les figures 6 et 9.

Des essais analogues aux précédents ont aussi été effectués sur des cellules de kératinocytes humains et sur des fibroblastes diploïdes de rat, avec des résultats identiquement bons.

Il est entendu que d'autres formes que celle des discoïdes pourraient convenir pour les microporteurs. On pourrait envisager des ellipses, des ovales, des carrés, des losanges, d'autres polygones, etc....

## Revendications

1. Support pour la culture de cellules ancrage-dépendantes (CAD), en forme de microporteurs en un matériau biocompatible, caractérisé en ce que les microporteurs présentent une géométrie bidimensionnelle et comportent deux faces d'ancrage apposées avec une épaisseur inférieure ou égale à 35 microns, faces sur chacune desquelles les cellules peuvent s'accrocher et se développer, sans pénétration possible des cellules entre ces deux faces.

2. Support suivant la revendication 1, caractérisé en ce que les microporteurs ont une épaisseur inférieure ou égale à 25 microns.

3. Support suivant la revendication 1, caractérisé en ce que les microporteurs ont une épaisseur inférieure ou égale à 10 microns.

4. Support suivant l'une des revendications 1 à 3, caractérisé en ce que les microporteurs ont une forme telle qu'obtenue par l'intersection orthogonale ou oblique de deux plans parallèles séparés l'un de l'autre d'une distance de 35 microns au maximum avec un corps plein.

5. Support suivant l'une des revendications 1 à 4, caractérisé en ce que les microporteurs ont, dans le milieu de culture, une densité comprise entre 0,90 et 1,10 g/cm³, de préférence entre 1,03 et 1,05 g/cm³.

6. Support suivant l'une des revendications 1 à 5, caractérisé en ce que les microporteurs sont transparents pour des ondes lumineuses, en particulier comprises entre 400 nm et 1000 nm.

7. Support suivant l'une des revendications 1 à 6, caractérisé en ce que les microporteurs sont revêtus sur leurs deux faces d'un mince dépôt métallique.

8. Support suivant la revendication 7, caractérisé en ce que le dépôt métallique est à base de titane.

9. Support suivant l'une des revendications 1 à 8, caractérisé en ce que les microporteurs comprennent une matière polymère.

10. Support suivant la revendication 9, caractérisé en ce que la matière polymère est du polystyrène.

11. Support suivant l'une des revendications 1 à 8, caractérisé en ce que les microporteurs comprennent un film hydrophile.

12. Support suivant la revendication 11, caractérisé en ce que le film hydrophile est en cellophane ou en poly-D-hydroxybutyrate.

13. Support suivant l'une quelconque des revendications 1 à 12, caractérisé en ce que les microporteurs présentent une biocompatibilité résultant d'un effet couronne sur leurs faces.

14. Procédé de fabrication d'un support pour la culture de cellules ancrage-dépendantes (CAD), en forme de microporteurs en un matériau biocompatible, caractérisé en ce qu'il comprend un découpage d'un film mince ou ultra-mince en microporteurs présentant une géométrie bidimensionnelle et comportant deux faces d'ancrage apposées avec une épaisseur inférieure ou égale à 35 microns, faces sur chacune desquelles les cellules peuvent s'accrocher et se développer, sans pénétration possible des cellules entre ces deux faces.

15. Procédé suivant la revendication 14, caractérisé en ce que le film mince a une épaisseur inférieure ou égale à 25 microns.

16. Procédé suivant la revendication 14, caractérisé en ce que le film mince a une épaisseur inférieure ou égale à 10 microns.

17. Procédé suivant l'une des revendications 14 à 16, caractérisé en ce qu'il comprend un découpage à l'aide d'un laser.

18. Procédé suivant l'une des revendications 14 à 16, caractérisé en ce qu'il comprend un découpage mécanique, à l'emporte-pièce.

19. Procédé suivant l'une quelconque des revendications 14 à 18, caractérisé en ce qu'il comprend un traitement du film pour le rendre biocompatible.

20. Procédé suivant la revendication 19, caractérisé en ce qu'il comprend, comme traitement préalable susdit, une application d'un mince dépôt métallique, en particulier à base de titane, sur chacune des surfaces du film mince, notamment par projection de plasma, ou respectivement par vaporisation.

21. Procédé suivant la revendication 19, caractérisé en ce qu'il comprend, comme traitement préalable susdit, un passage d'une décharge électrique à effet couronne sur le film mince ou ultra-mince.

## Claims

1. A support for the culture of anchorage dependent cells (ADC's) in the shape of microcarriers of a biocompatible material, characterized in that the microcarriers have a two-dimensional geometry and comprise two apposed anchorage surfaces, with a thickness smaller than or equal to 35 microns, both of said surfaces enablING the cells to attach themselves and to develop without any possibility for the cells to penetrate between these two surfaces.

2. A support according to claim 1, characterized in that the microcarriers have a thickness smaller than or equal to 25 microns.

3. A support according to claim 1, characterized in that the microcarriers have a thickness smaller than or equal to 10 microns.

4. A support according to any one of the claims 1 to 3, characterized in that the microcarriers have a shape such as obtained by orthogonal or oblique intersection of two parallel planes at a mutual distance of 35 microns at the most with a solid.

5. A support according to any one of the claims 1 to 4, characterized in that the microcarriers have, in the culture medium, a density comprised between 0.90 and 1.10 g/cm³, and preferably between 1.03 and 1.05 g/cm³.

6. A support according to any one of the claims 1 to 5, characterized in that the microcarriers are transparent for light waves comprised between 400 nm and 1000 nm.

7. A support according to any one of the claims 1 to 6, characterized in that the microcarriers are coated onto their two surfaces with a thin metallic deposit.

8. A support according to claim 7, characterized in that the metallic deposit is a titanium deposit.

9. A support according to any one of the claims 1 to 8, characterized in that the microcarriers comprise a polymeric material.

10. A support according to claim 9, characterized in that the polymeric material is polystyrene, polycarbonate or polyterephthalate.

11. A support according to any one of the claims 1 to 8, characterized in that the microcarriers comprise a hydrophilic film.

12. A support according to claim 11, characterized in that the hydrophilic film is of cellophane or of poly-D-hydroxybutyrate.

13. A support according to any one of claims 1 to 12, characterized in that the microcarriers have a biocompatibility resulting from a corona effect on their surfaces.

14. A method for manufacturing a support for the culture of anchorage dependent cells (ADC's) in the shape of microcarriers of a biocompatible material, characterized in that it comprises cutting a thin or ultra-thin film into microcarriers having a two-dimensional geometry and comprising two apposed anchorage surfaces, with a thickness smaller than or equal to 35 microns, both of said surface enabling the cells to attach themselves and to develop without any possibility for the cells to penetrate between these two surfaces.

15. A method according to claim 14, characterized in that the thin film has a thickness smaller than or equal to 25 microns.

16. A method according to claim 14, characterized in that the film has a thickness smaller than or equal to 10 microns.

17. A method according to any one of the claims 14 to 16, characterized in that it comprises a cutting by means of a laser.

18. A method according to any one of the claims 14 to 16, characterized in that it comprises a mechanical cutting by punching.

19. A method according to any one of claims 14 to 18, characterized in that it comprises a treatment of the film in order to render it biocompatible.

20. A method according to claim 19, characterized in that it comprises, as said previous treatment, applying a thin metallic deposit, in particular a titanium deposit, on each of the surfaces of the thin film, more particularly by plasma projection or respectively by vaporization.

21. A method according to claim 19, characterized in that it comprises, as said previous treatment, passing an electrical corona discharge on the thin or ultra-thin film.

## Patentansprüche

1. Unterlage für die Kultivierung von Haftungs-abhängigen Zellen (CAD) in Form von Mikroträgern aus einem biokompatiblen Material, dadurch gekennzeichnet, daß die Mikroträger eine zweidimensionale Geometrie besitzen und zwei aneinanderliegende Flächen zur Anhaftung aufweisen mit einer Stärke von 35 »m oder weniger, wobei sich auf jeder der beiden Flächen die Zellen anheften und entwickeln können, ohne daß ein Eindringen der Zellen zwischen die beiden Flächen möglich ist.

2. Unterlage nach Anspruch 1, dadurch gekennzeichnet, daß die Mikroträger höchstens 25 »m dick sind.

3. Unterlage nach Anspruch 1, dadurch gekennzeichnet, daß die Mikroträger höchstens 10 »m dick sind.

4. Unterlage nach einem der Ansprüche 1 bis 3, dadurch gekennzeichnet, daß die Mikroträger eine Form besitzen, die erhältlich ist durch orthogonale oder schräge Schnitte durch zwei parallele Flächen, die durch einen massiven Körper in einem Abstand von maximal 35 »m voneinander getrennt sind.

5. Unterlage nach einem der Ansprüche 1 bis 4, dadurch gekennzeichnet, daß die Mikroträger im Kulturmedium eine Dichte im Bereich von 0,90 bis 1,10 g/cm³, vorzugsweise von 1,03 bis 1,05 g/cm³, aufweisen.

6. Unterlage nach einem der Ansprüche 1 bis 5, dadurch gekennzeichnet, daß die Mikroträger durchlässig sind für Lichtwellen, insbesondere für solche mit Wellenlängen zwischen 400 nm und 1000 nm.

7. Unterlage nach einem der Ansprüche 1 bis 6, dadurch gekennzeichnet, daß die Mikroträger auf ihren beiden Flächen mit einer dünnen metallischen Schicht überzogen sind.

8. Unterlage nach Anspruch 7, dadurch gekennzeichnet, daß die metallische Schicht auf Titanbasis ist.

9. Unterlage nach einem der Ansprüche 1 bis 8, dadurch gekennzeichnet, daß die Mikroträger ein Polymermaterial enthalten.

10. Unterlage nach Anspruch 9, dadurch gekennzeichnet, daß das Polymermaterial Polystyrol ist.

11. Unterlage nach einem der Ansprüche 1 bis 8, dadurch gekennzeichnet, daß die Mikroträger einen hydrophilen Film umfassen.

12. Unterlage nach Anspruch 11, dadurch gekennzeichnet, daß der hydrophile Film aus Cellophan oder aus Poly-D-hydroxybutyrat ist.

13. Unterlage nach einem der Ansprüche 1 bis 12, dadurch gekennzeichnet, daß die Mikroträger aufgrund eines Koronaeffektes auf ihren Flächen biokompatibel sind.

14. Verfahren zur Herstellung einer Unterlage zur Kultivierung Haftungs-abhängiger Zellen (CAD) in Form von Mikroträgern aus einem biokompatiblem Material, dadurch gekennzeichnet, daß ein dünner oder ultradünner Film in Mikroträger zerschnitten wird, die eine zweidimensionale Geometrie besitzen und zwei aneinanderliegende Flächen aufweisen mit einer Stärke von 35 »m oder weniger, wobei sich auf jeder der beiden Flächen die Zellen anheften und entwickeln können, ohne daß ein Eindringen der Zellen zwischen die beiden Flächen möglich ist.

15. Verfahren nach Anspruch 14, dadurch gekennzeichnet, daß der dünne Film höchstens 25 »m dick ist.

16. Verfahren nach Anspruch 14, dadurch gekennzeichnet, daß der dünne Film höchstens 10 »m dick ist.

17. Verfahren nach einem der Ansprüche 14 bis 16, dadurch gekennzeichnet, daß das Zerschneiden mit Hilfe eines Lasers erfolgt.

18. Verfahren nach einem der Ansprüche 14 bis 16, dadurch gekennzeichnet, daß das Zerschneiden mechanisch mittels einer Stanze erfolgt.

19. Verfahren nach einem der Ansprüche 14 bis 18, dadurch gekennzeichnet, daß es eine Behandlung des Films umfaßt, um diesen biokompatibel zu machen.

20. Verfahren nach Anspruch 19, dadurch gekennzeichnet, daß die obengenannte vorangehende Behandlung das Aufbringen einer dünnen Metallschicht, insbesondere auf Titanbasis, auf jeder der Oberflächen des dünnen Filmes umfaßt, insbesondere durch Plasmaauftrag, beziehungsweise durch Verdampfen.

21. Verfahren nach Anspruch 19, dadurch gekennzeichnet, daß die obengenannte vorangehende Behandlung die Durchführung einer elektrischen Entladung mit Koronaeffekt auf dem dünnen oder ultradünnen Film umfaßt.
